# EUROPEAN PATENT APPLICATION

(11) **EP 0 847 774 A2**
(43) Date of publication of application: **17.06.1998**
(21) Application number: 97121143.8
(22) Date of filing: 02.12.1997
(51) Int. Cl.: A61N 1/16

(54) **Multilayer panel for shielding from the influences of geopathogenic regions**

(30) Priority: 10.12.1996 IT PD960305
(71) Applicant: Bonomo, Dario, 35042 Este (Padova) (IT)
(72) Inventor: Bonomo, Dario, 35042 Este (Padova) (IT)
(74) Representative: Modiano, Guido, Dr.-Ing.

(57) **Abstract**

A multilayer panel particularly for shielding from the influences of geopathogenic regions, the panel (10) comprising at least two mutually opposite outer layers (11) made of geotextile material, inside which at least one layer (12) of pressed cork assembled by using microwaves, at least one aluminum lamination (13), and at least one copper grid (14) with a preset mesh are associated.

## Description

The present invention relates to a multilayer panel particularly for shielding from the influences of geopathogenic regions.

Many studies are currently aimed at detecting and studying environmental influences on human disorders.

In particular, a recently created discipline commonly known as "geobiology" studies and researches the environmental characteristics and the geological influences affecting human physiological life.

The planet Earth is currently considered an organized living entity, in the so-called "Gaia hypothesis", which is crossed by telluric currents which are sometimes unfavorable to the human being.

Studies have in fact found, on the surface of the earth, regions that produce negative influences on human health.

These regions are classified as "geopathogenic" and the consequent diseases, which depend on the place where one lives or works, are known as "geopathies".

Merely by way of example, the following can be considered as factors that cause earth-generated magnetic anomalies: underground water streams, the Hartmann grid, the natural electromagnetic grid, the Curry grid.

In particular, underground water streams generate an electric current, termed ionic current, owing to electric potentials of the boundary surfaces.

Natural radiation is modified by said ionic current, producing the anomalies that are typical of geopathogenic regions.

The Hartmann grid or natural electromagnetic grid is instead a system of permanent waves which are part of terrestrial magnetism.

Merely by way of information, its grid covers the entire Earth and runs from north to south and from east to west with a spacing of approximately 2 x 2.5 meters.

The Curry grid is an electrical grid and is generated from below but acts as if it were originated from above.

It is arranged diagonally and forms a 45° angle with respect to the north-south axis.

Recent studies have shown that it has a spacing of approximately 3.5 x 3.5 meters.

The aim of the present invention is to provide a multilayer panel which is suitable to shield from the influences of geopathogenic regions of the surface of the earth in which anomalies or alterations in the ordinary radiation and in the normal magnetic field occur.

Within the scope of this aim, an object of the present invention is to provide a panel which can be applied particularly in the building field to regulate environmental waves.

Another object of the present invention is to provide a panel whose application or use is particularly simple and safe for the user.

Another object of the present invention is to provide a panel which has no collateral contraindication as regards human health.

Another object of the present invention is to provide a panel whose costs are affordable even for individuals having no particular financial resources.

Another object of the present invention is to provide a panel which can be manufactured with conventional technologies and optionally with economies of scale.

This aim, these objects and others which will become apparent hereinafter are achieved by a multilayer panel particularly for shielding from the influences of geopathogenic regions, characterized in that it comprises, between at least two layers of geotextile material, at least one first pressed cork panel assembled by using microwaves, at least one first aluminum lamination and at least one copper grid having a mesh of a preset size.

Further characteristics and advantages of the present invention will become apparent from the description of some embodiments thereof, illustrated by way of non-limitative example in the accompanying drawings, wherein:
figure 1 is an exploded view of a panel according to the invention in a first embodiment;
figure 2 is a sectional orthographic projection view of the panel of figure 1;
figure 3 is another orthographic projection view of the panel of figure 1;
figure 4 is an exploded view of a multilayer panel according to the invention in a second embodiment;
figure 5 is a sectional orthographic projection view of the panel of figure 4;
figure 6 is another orthographic projection view of the panel of figure 4;
figure 7 is an exploded view of a multilayer panel according to the invention in a third embodiment;
figure 8 is a sectional orthographic projection view of the panel of figure 7;
figure 9 is another orthographic projection view of the panel of figure 7.

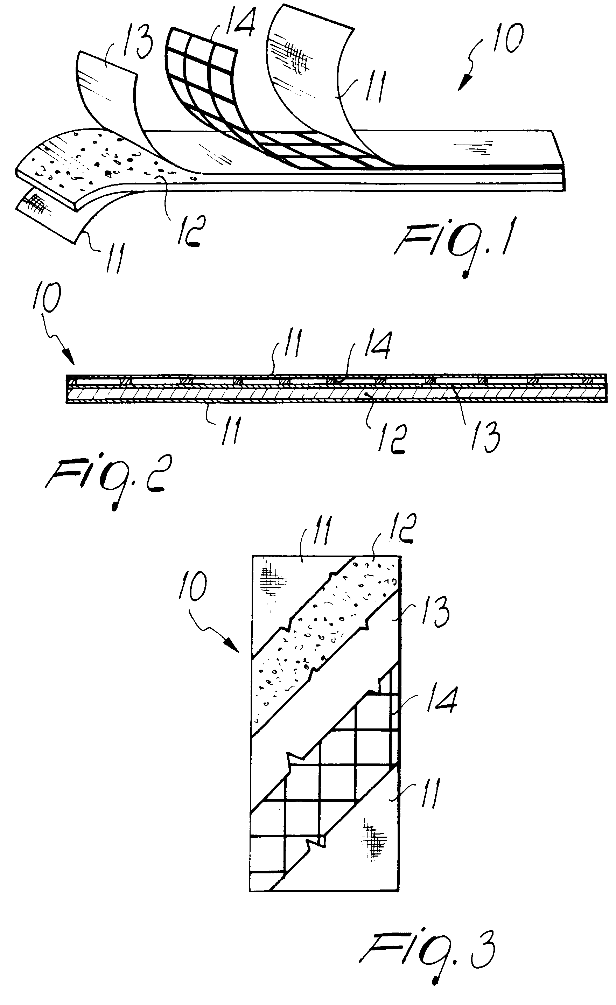

With particular reference to figures 1 to 3, a multilayer panel particularly for shielding from geopathogenic influences, according to the invention, is generally designated by the reference numeral 10 in a first embodiment.

The panel 10 comprises the following components, packed in a sandwich-like arrangement between two outer layers of geotextile or other protective material designated by the reference numeral 11: a first panel 12, made of pressed cork and assembled by using microwaves; an aluminum lamination or band 13 (which has a reflective function); and a copper grid 14 with a mesh having a preset spacing which is in any case not greater than the average of 0.4 x 0.4 meters.

The above-described panel is particularly suitable for use in the building field, arranged for example below the floor foundation and connected to other panels of the same kind before laying the flooring.

It is recommended whenever building work is performed in regions affected by particularly significant telluric waves and in any case whenever ambient waves are to be regulated.

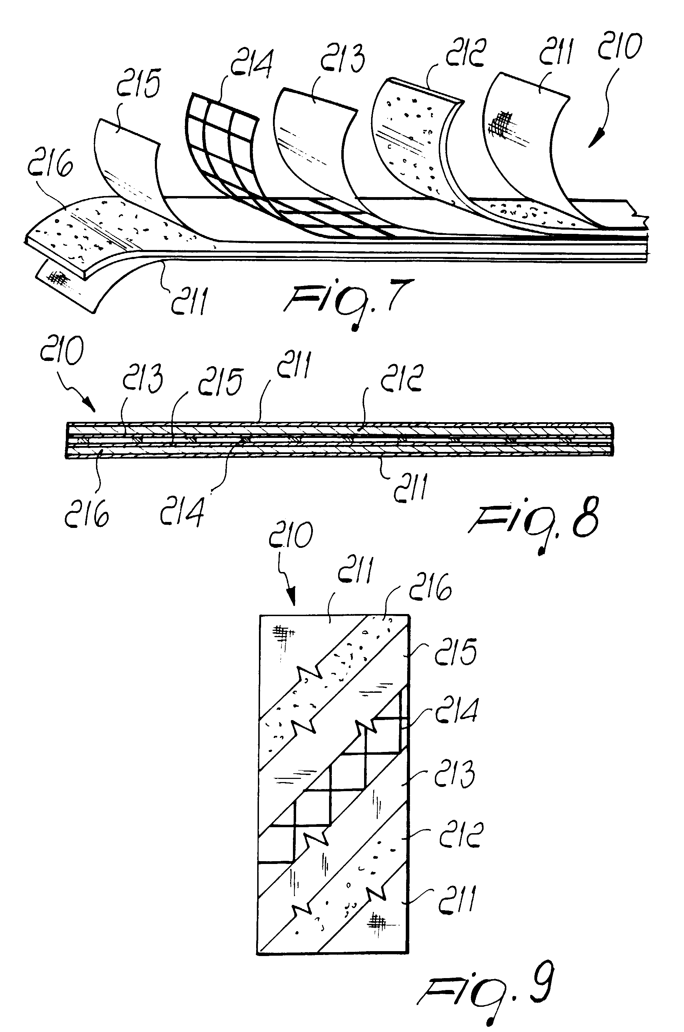

With particular reference to figures 4 to 7, a multilayer panel particularly for shielding from the influences of geopathogenic regions, according to the invention, is generally designated by the reference numeral 110 in a second embodiment.

The panel 110 comprises, between two outer layers made of biocompatible fabric, both of which are designated by the reference numeral 111: a first layer 112 made of cork assembled by using microwaves; an aluminum lamination, designated by the reference numeral 113; and a copper grid 114, whose mesh has a preset spacing which in any case does not exceed an average of 0.25 x 0.25 meters.

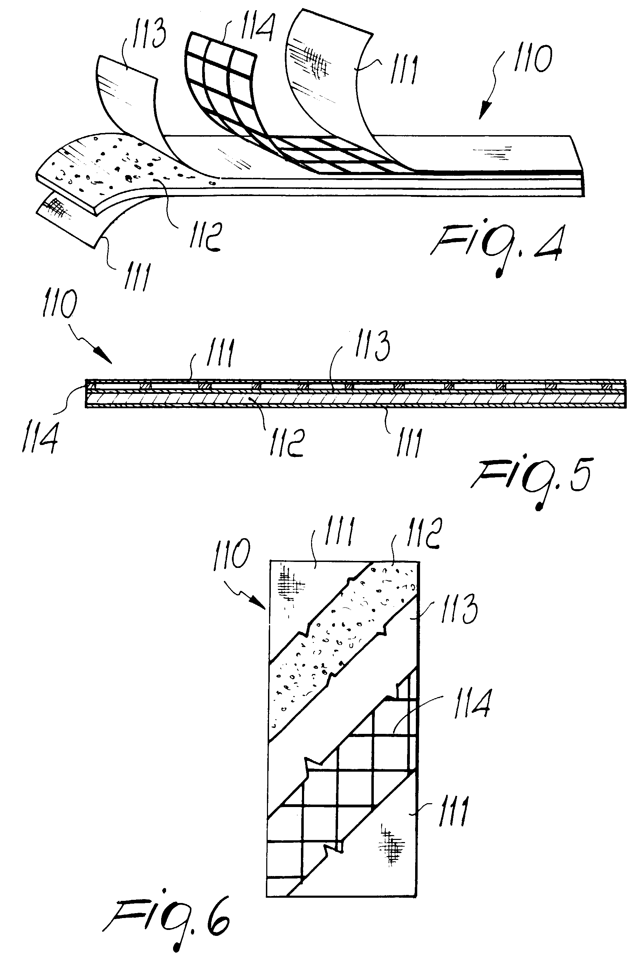

With particular reference to figures 6 to 9, a multilayer panel, particularly for shielding from geopathogenic influences, according to the invention, is generally designated by the reference numeral 200 in a third embodiment.

The panel 210 comprises the following components, packed in a sandwich-like arrangement between two outer layers made of biocompatible fabric and designated by the reference numeral 211: a first panel 212, made of pressed cork assembled by using microwaves; a first aluminum lamination or band 213; a copper grid 214 with a mesh having a preset spacing which in any case does not exceed the average of 0.25 x 0.25 meters; a second aluminum lamination 215; and a second lamination 216 again made of pressed cork assembled by using microwaves.

Merely by way of example, the panels of the second and third embodiments, which shield from the negative influences caused by alterations in magnetic fields and radiant fields of the earth can be arranged in the bed, under the mattress or in ready-made mattresses constituted by multiple layers of wool, latex, coconut fiber and/or the like.

The use of the panels according to the invention is necessary whenever the bed is in a room built on a site through which particularly significant telluric waves pass; in any case, when it is used it is found to be an excellent regulator of ambient waves even in situations that are not particularly severe.

In practice it has been observed that the present invention has achieved the intended aim and objects.

In particular, it should be noted that the multilayer panel according to the invention substantially fully solves the problem of shielding the human being from geopathogenic influences caused by alterations in the magnetic or radiant field of the earth.

It should also be noted that the panel according to the invention has a structure which is particularly flexible in terms of its fields of application, which can be the most disparate.

Attention is also drawn to the ease of use of the panel according to the invention, which is not difficult to use and has no contraindications for users.

It should also be noted that the simple structure of the panel according to the invention causes it to have fairly low costs which can be afforded even by families having modest incomes.

The present invention is susceptible of numerous modifications and variations, all of which are within the scope of the inventive concept; likewise, the details may be replaced with other technically equivalent elements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A multilayer panel particularly for shielding from geopathogenic influences, characterized in that it comprises, between at least two outer layers made of geotextile material, at least one first layer made of pressed cork assembled by using microwaves, at least one aluminum lamination and at least one copper grid with a preset mesh.

2. A multilayer panel particularly for shielding from geopathogenic influences, characterized in that it comprises, between at least two outer layers made of biocompatible fabric, at least one first layer made of pressed cork assembled by using microwaves, at least one first aluminum lamination, and at least one copper grid with a preset mesh.

3. A panel according to claim 2, characterized in that it comprises at least one second aluminum lamination.

4. A panel according to claim 2, characterized in that it comprises at least one second layer made of pressed cork and assembled by using microwaves.
